(19) Europäisches Patentamt
European Patent Office
Office européen des brevets

(11) Publication number: 0 219 307 A2

# (12) EUROPEAN PATENT APPLICATION

(21) Application number: 86307779.8

(22) Date of filing: 08.10.86

(51) Int. Cl.[4]: **C 07 D 215/14**
C 07 D 215/18, A 61 K 31/47

(30) Priority: 16.10.85 US 788180

(43) Date of publication of application: 22.04.87 Bulletin 87/17

(84) Designated Contracting States: AT BE CH DE FR GB IT LI LU NL SE

(71) Applicant: **MERCK FROSST CANADA INC.**
**16711 Trans-Canada Highway**
**Kirkland Quebec (CA)**

(72) Inventor: **Young, Robert N.**
**216 Senneville**
**Quebec H9X 3L2 (CA)**

**Zamboni, Robert**
**233 Boul.Jacques Cartier Est**
**Longueuil Quebec J4L 1E1 (CA)**

**Leger, Serge**
**51 Lamarche**
**Dollard des Ormeaux (CA)**

(74) Representative: **Crampton, Keith John Allen et al**
**D YOUNG & CO 10 Staple Inn**
**London WC1V 7RD (GB)**

## (54) 2-Substituted quinolines.

(57) Novel compounds have the formula:

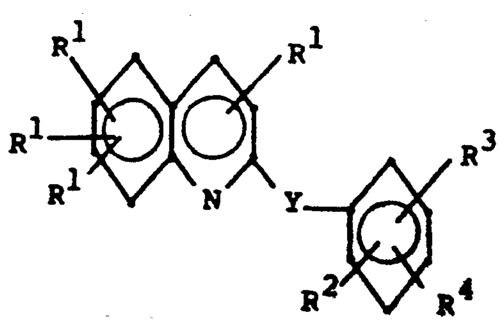

or are pharmaceutically acceptable salts thereof. In the formula, Y is $-(CR^2=CR^2)_n-$ or $-(C\equiv C)_n-$;
$R^1$ is H, halogen, $C_1$-$C_8$ alkyl, $C_2$-$C_8$ alkenyl, $C_2$-$C_8$ alkynyl, $-CF_3$, $OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, substituted or unsubstituted phenyl, substituted or unsubstituted benzyl, or substituted or unsubstituted phenethyl;
$R^2$ is H, $C_1$ -$C_8$ alkyl, $C_2$-$C_8$ alkenyl, $C_2$-$C_8$ alkynyl, $-CF_3$, substituted or unsubstituted phenyl, substituted or unsubstituted benzyl, or substituted or unsubstituted phenethyl;
$R^3$ is $-(A)_m-(CR^2R^2)_m-(CR^2R^4)_m-CR^2R$ , with the proviso that $R^3$ is not CHO when it is para to Y;
$R^4$ is H, halogen, $-NO_2$, $-OR^2$, $-SR^2$, $NR^2R^2$, or $C_1$-$C_8$ alkyl, or $(R^4)_2$ is O=;
A is =C=O or $CR^2(OR^6)$;
$R^6$ is H, $C_1$-$C_6$ alkyl, $-(C=O)R^7$, unsubstituted phenyl or unsubstituted benzyl;
$R^7$ is H, $C_1$-$C_8$ alkyl, $C_2$-$C_8$ alkenyl, $C_2$-$C_8$ alkynyl, $-CF_3$, or unsubstituted phenyl, benzyl, or phenethyl;
each m is 0 or integer of from 1 to 8, provided that at least one m is not 0;
and n is 1 or 2. The compounds are antagonists of leukotrienes and inhibitors of leukotriene biosynthesis. These compounds are useful as anti-asthmatic, anti-allergic, anti-inflammatory and cytoprotective agents.

EP 0 219 307 A2

**Description**

## 2-SUBSTITUTED QUINOLINES

This invention relates to compounds that act as antagonists of the leukotrienes and as inhibitors of their biosynthesis, and to their preparation and use, inter alia in pharmaceutical formulations.

The leukotrienes and their biological activities, especially their roles in various disease states and conditions have been described (see, for example, European Patent Specification EP-A-0 140 648. Several classes of compounds antagonize the action of leukotrienes in mammals, especially humans. See for example: United Kingdom Patent Specifications GB-A-2 058 785 and GB-A-2 094 301; and European Patent Specification EP-A-0 056 172, EP-A-0 061 800 and EP-A-0 068 739.

EP-A-0 110 405 describes anti-inflammatory and anti-allergic substituted benzenes which are disclosed to be leukotriene inhibitors, i.e., inhibitors of the 5-lipoxygenase pathway.

The compounds of this invention have Formula I:

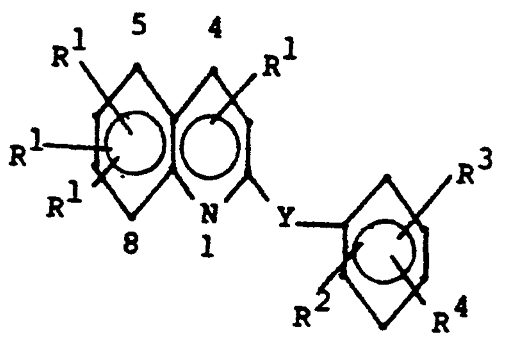

I .

in which:

Y is $-(CR^2=CR^2)_n-$ or $-(C\equiv C)_n-$;

$R^1$ is H, halogen, $C_1$-$C_8$ alkyl, $C_2$-$C_8$ alkenyl, $C_2$-$C_8$ alkynyl, $-CF_3$, $OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, substituted or unsubstituted phenyl, substituted or unsubstituted benzyl, or substituted or unsubstituted phenethyl;

$R^2$ is H, $C_1$-$C_8$ alkyl, $C_2$-$C_8$ alkenyl, $C_2$-$C_8$ alkynyl, $-CF_3$, substituted or unsubstituted phenyl, substituted or unsubstituted benzyl, or substituted or unsubstituted phenethyl;

$R^3$ is $-(A)_m-(CR^2R^2)_m-(CR^2R^4)_m-CR^2R$ , with the proviso that $R^3$ is not CHO when it is para to Y;

$R^4$ is H, halogen, $-NO_2$, $-OR^2$, $-SR^2$, $NR^2R^2$, or $C_1$-$C_8$ alkyl, or $(R^4)_2$ is $O=$;

A is $=C=O$ or $CR^2(OR^6)$;

$R^6$ is H, $C_1$-$C_6$ alkyl, $-(C=O)R^7$, unsubstituted phenyl or unsubstituted benzyl;

$R^7$ is H, $C_1$-$C_8$ alkyl, $C_2$-$C_8$ alkenyl, $C_2$-$C_8$ alkynyl, $-CF_3$, or unsubstituted phenyl, benzyl, or phenethyl;

each m is 0 or integer of from 1 to 8, provided that at least one m is not O;

and n is 1 or 2; provided that where two or more of any variable are present, each is the same as or different from the other(s);

or are pharmaceutically acceptable salts of such components.

Alkyl, alkenyl, and alkynyl include linear, branched, and cyclic structures. Thus, alkyl would include, for example n-butyl, sec-butyl, tert-butyl and cyclobutyl.

Substituted phenyl, benzyl, and phenethyl mean those having 1 or 2 substitutents on the benzene ring selected from $C_1$-$C_6$ alkyl, $R^5$, $NO_2$, $SCF_3$, halogen, $-COR^5$, $COR^7$, CN, and $CF_3$, where $R^5$ is $-OR^6$, $-SR^6$, or $NR^6R^6$.

Halogen includes F, Cl, Br and I.

It is intended that the definitions of any variable (e.g., $R^1$, $R^2$ or m) in a particular molecule is independent of its definitions elsewhere in the molecule. Thus, $-NR^2R^2$ represents inter alia $-NH_2$, $-NHCH_3$ and $-NHC_6H_5$.

Some of the compounds of the present invention contain one or more centres of asymmetry and may thus give rise to diastereoisomers and optical isomers. The present invention comprehends such possible diastereoisomers as well as their racemic and resolved optically active forms.

Some of the compounds described herein contain olefinic double bonds and, unless specified otherwise, include both E and Z geometric isomers.

One group of preferred compounds represented by Formula Ia:

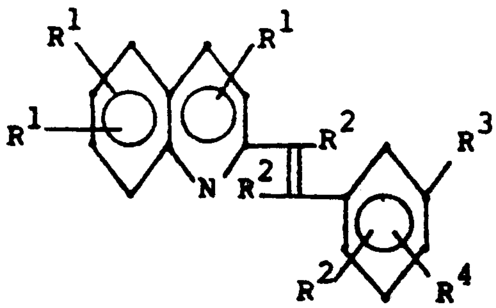

Ia

where $R^1$ is H, halogen, $CH_3$, $-CF_3$, or $SCF_3$; $R^2$ is H, $C_1$-$C_3$ alkyl, $C_2$-$C_3$ alkenyl, or $-CF_3$; and $R^3$ to $R^7$, m, and A are as defined for Formula I; and their pharmaceutically acceptable salts.

Other preferred compounds of formula I are represented by Formula Ib:

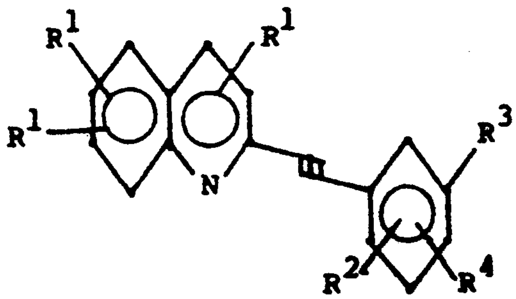

Ib

where $R^1$ is H, halogen, $CH_3$, or $SCF_3$; $R^2$ is H, $C_1$-$C_3$ alkyl, $C_2$-$C_3$ alkenyl, or $CF_3$; and $R^3$ to $R^7$, m and A are as defined for Formula I; and their pharmaceutically acceptable salts.

The pharmaceutically acceptable salts are those prepared from pharmaceutically acceptable non-toxic acids including inorganic acids and organic acids. Such acids include acetic, benzenesulphonic, benzoic, camphorsulphonic, citric, ethanesulphonic, fumaric, gluconic, glutamic, hydrobromic, hydrochloric, isethionic, lactic, maleic, malic, mandelic, methanesulphonic, mucic, nitric, panoic, pantothenic, phosphoric, succinic, sulphuric, tartaric and p-toluenesulphonic acids. Particularly preferred are hydrobromic, hydrochloric, phosphoric and sulphuric acids.

Compounds of Formula I, including their pharmaceutically acceptable salts and lactone, lactam or thiolactam forms, have been found active as antagonists of SRS-A and especially of leukotriene $D_4$. These compounds also have modest inhibitory activity on leukotriene biosynthesis but are primarily of therapeutic interest as antagonists. The activity of the compounds of Formula I can be detected and evaluated by known methods (see, for example, US Patent Specification US-A-4 296 129).

Because of their activity as leukotriene antagonists or inhibitors, compounds of the present invention are useful as anti-asthmatic, anti-allergic, and anti-inflammatory agents and are useful in treating allergic rhinitis and chronic bronchitis and for amelioration of skin diseases such as psoriasis and atopic eczema. The compounds are also useful to antagonize or inhibit the pathologic actions of leukotrienes on the cardiovascular and vascular systems, which may , for example, result in angina. Compounds of the present invention have also been found useful in the treatment of inflammatory and allergic diseases of the eye, including allergic conjunctivitis, and as cytoprotective agents.

Thus, such compounds may also be used to treat or prevent mammalian (especially, human) disease states such as erosive gastritis; erosive esophagitis; inflammatory bowel disease; ethanol-induced haemorrhagic erosions; hepatic ischemic; noxious-agent-induced damage or necrosis of hepatic, pancreatic, renal, or myocardial tissue; liver parenchymal damage caused by hepatoxic agents such as $CCl_4$ and D-galactosamine; ischemic renal failure; disease-induced hepatic damage; bile-salt-induced pancreatic or gastric damage; trauma- or stress-induced cell damage; and glycerol-induced renal failure. Such compounds are also valuable in the prevention and treatment of other such disease states in which the leukotrienes are the causative factor, e.g. obstructive airway diseases such as allergic bronchial asthma.

The cytoprotective activity of a compound may be observed in both animals and man by noting the increased resistance of the gastrointestinal mucosa to the noxious effects of strong irritants, for example, the ulcerogenic effects of aspirin or indomethacin. In addition to lessening the effect of non-steroidal anti-inflammatory drugs on the gastrointestinal tract, animal studies show that cytoprotective compounds will prevent gastric lesions induced by oral administration of irritants such as strong acids, strong bases, ethanol and hypertonic saline solutions.

Two assays can be used to measure cytoprotective ability. These assays are (A) an ethanol-induced lesion assay and (B) and indomethacin-induced ulcer assay and are described in European Patent Specification EP-A-0 140 684.

The magnitude of a prophylactic or therapeutic dose of a compound of Formula I will, of course, vary with the nature of the severity of the condition to be treated and with the particular compound of Formula I and its route of administration. It will also vary according to the age, weight and response of the individual patient. In general, the daily dose range for anti-asthmatic. anti-allergic or anti-inflammatory use and generally, uses other than cytoprotection, lie within the range of from 0.001 mg to 100 mg per kg body weight of a mammal, preferably 0.01 mg to 10 mg per kg, and especially 0.1 to 1 mg per kg, in single or divided doses. On the other hand, it may be necessary to use dosages outside these limits in some cases.

The exact amount of a compound of the Formula I to be used as a cytoprotective agent will depend on, inter alia, whether it is being administered to heal damaged cells or to avoid future damage, on the nature of the damaged cells (e.g., gastrointestinal ulcerations or nephrotic necrosis), and on the nature of the causative agent. An example of the use of a compound of the invention in avoiding future damage would be co-administration with a non-steroidal anti-inflammatory drug that might otherwise cause such damage (for example, indomethacin). For such use, the compound of Formula I is administered from 30 minutes before to 30 minutes after administration of the NSAID. Preferably it is administered before or simultaneously with the NSAID, for example, in a combined dosage form.

The effective daily dosage level for compounds of Formula I inducing cytoprotection in mammals, especially humans, will generally range from 0.1 mg/kg to 100 mg/kg, preferably 1 mg/kg to 100 mg/kg. The dosage may be administered in single or divided individual doses, and by any suitable route of administration, for example, oral, rectal, topical, parenteral (including subcutaneous, intramuscular, and intravenous), ocular (ophthalmic), pulmonary (nasal or buccal inhalation), or nasal administration, although the most suitable route in any given case will depend on the nature and severity of the conditions being treated and on the nature of the active ingredient.

For intravenous administration, a suitable dosage range for anti-asthmatic, anti-inflammatory or anti-allergic use is from 0.001 mg to 10 mg (preferably from 0.01 mg to 1 mg) of a compound of Formula I per kg of body weight per day and for cytoprotective use from 0.1 mg to 100 mg (preferably from 1 mg to 100 mg and especially from 1 mg to 10 mg) of a compound of Formula I per kg of body weight per day.

In the case of an oral composition, a suitable dosage range for anti-asthmatic, anti-inflammatory or anti-allergic use is, e.g. from 0.01 mg to 100 mg of a compound of Formula I per kg of body weight per day, preferably from 0.1 mg to 10 mg per kg, and for cytoprotective use is from 0.1 mg to 100 mg (preferably from 1 mg to 100 mg and especially from 10 mg to 100 mg) of a compound of Formula I per kg of body weight per day.

For administration by inhalation, the compounds of the present invention are conveniently delivered in the form of an aerosol spray from pressurized packs or a nebuliser, or as a powder which may be formulated as a cartridge from which the powder composition may be inhaled with the aid of a suitable device. The preferred delivery system for inhalation is a metered dose inhalation (MDI) aerosol, which may be formulated as a suspension or solution in fluorocarbon propellants.

Suitable topical formulations of Compound I include transdermal devices, aerosols, creams, ointments, lotions and dusting powders.

For the treatment of diseases of the eye, ophthalmic preparations for ocular administration comprising 0.001 -1% by weight solutions or suspensions of the compounds of Formula I in an acceptable ophthalmic formulation may be used.

In practical use, the compounds of Formula I can be combined as the active ingredient in intimate admixture with a pharmaceutical carrier and optionally other therapeutic ingredients according to conventional pharmaceutical compounding techniques. The carrier may take a wide variety of forms depending on the form of preparation desired for administration, e.g., oral or parenteral (including intravenous). In preparing the compositions for oral dosage form, any pharmaceutical media may be used, for example, water, glycols, oils, alcohols, flavoring agents, preservatives and coloring agents in the case of oral liquid preparations, such as suspensions, elixirs and solutions; or carriers such as starches, sugars, microcrystalline cellulose, diluents, granulating agents, lubricants, binders and disintegrating agents in the case of oral solid preparations such as powders, capsules and tablets, with solid oral preparations being preferred to liquid preparations. Because of their ease of administration, tablets and capsules represent the most advantageous oral dosage unit form. If desired, tablets may be coated by standard aqueous or non-aqueous techniques.

In addition to the common dosage forms set out above, the compounds of Formula I may also be administered by controlled release means and/or delivery devices such as those described in U. S. Patent Specifications US-A-3 845 770, US-A-3 916 899; US-A-3 536 809; US-A-3 598 123; US-A-3 630 200 and US-A-4 008 719.

Pharmaceutical compositions of the present invention suitable for oral administration may be presented as discrete units such as capsules, cachets or tablets each containing a predetermined amount of the active ingredient, as a powder or granules or as a solution or a suspension in an aqueous liquid, a non-aqueous liquid, an oil-in-water emulsion or a water- in-oil liquid emulsion. Such compositions may be prepared by any of the methods of pharmacy but all methods include the step of bringing into association the active ingredient with the carrier which is constituted of one or more necessary ingredients. In general, the compositions are prepared by uniformly and intimately admixing the active ingredient with liquid carriers or finely divided solid

carriers or both, and then, if necessary, shaping the product into the desired presentation. For example, a tablet may be prepared by compression or molding, optionally with one or more accessory ingredients. Compressed tablets may be prepared by compressing in a suitable machine, the active ingredient in a free-flowing form such as powder or granules, optionally mixed with a binder, lubricant, inert diluent, surfactant or dispersing agent. Molded tablets may be made by molding in a suitable machine, a mixture of the powdered compound moistened with an inert liquid diluent. Desirably, each tablet contains from 2.5 mg to 500 mg of the active ingredient and each cachet or capsule contains from 2.5 to 500 mg of the active ingredient.

The following are examples of representation pharmaceutical dosage forms for the compounds of Formula I:

| Injectable Suspension (I.M.) | mg/ml |
|---|---|
| Compound of Formula I | 10 |
| Methylcellulose | 5.0 |
| Polysorbate 80 | 0.5 |
| Benzyl alcohol | 9.0 |
| Benzalkonium chloride | 1.0 |
| Water for injection to a total volume of 1 ml. | |

| Tablet | mg/tablet |
|---|---|
| Compound of Formula I | 25 |
| Microcrystalline Cellulose | 415 |
| Povidone | 14.0 |
| Pregelatinized Starch | 43.5 |
| Magnesium Stearate | 2.5 |
| | 500 |

| Capsule | mg/capsule |
|---|---|
| Compound of Formula I | 25 |
| Lactose Powder | 573.5 |
| Magnesium Stearate | 1.5 |
| | 600 |

In addition to the compounds of Formula I, the pharmacuetical compositions of the present invention can also contain other active ingredients, such as cyclooxygenase inhibitors, non-steroidal anti- inflammatory drugs (NSAIDs), peripheral analgesic agents such as zomepirac and diflunisal. The weight ratio of the compound of the Formula I to the second active ingredient may be varied and will depend upon the effective dose of each ingredient. Generally, an effective dose of each will be used. Thus, for example, when a compound of Formula I is combined with an NSAID the weight ratio of the compound Formula I to the NSAID will generally range from 1000:0 to 1:1000. Combinations of a compound of Formula I and other active ingredients will generally also be within the aforementioned range, but in each case, an effective dose of each active ingredient should be used.

NSAIDs can be characterized into five groups, viz. (1) propionic acid derivatives; (2) acetic acid derivatives; (3) fenamic acid derivatives; (4) biphenylcarboxylic acid derivatives; and (5) oxicams, including their pharmaceutically acceptable salts. NSAIDs that can be used in accordance with this invention are those disclosed in European Patent Specification EP-A-0 140 684.

Pharmaceutical compositions comprising the Formula I compounds may also contain inhibitors of the

biosynthesis of the leukotrienes such as are disclosed in European Patent Specifications EP-A-0 138 481, EP-A-0 115 394, EP-A-0 136 893 and EP-A-0 140 709, and leukotriene antagonists such as those disclosed in EP-A-0 106 565, EP-A-0 104 885, EP-A-0 056 172, EP-A-0 061 800 and UK Patent Specification GB-A-2 058 785.

Pharmaceutical compositions comprising the Formula I compounds may also contain as the second active ingredient prostaglandin antagonists such as those disclosed in EP-A-0 011 067 or thromboxane antagonists such as those disclosed in US Patent Specification US-A-4 237 160. They may also contain histidine decarboxylase inhibitors such as α-fluoromethyl-histidine, described in US-A-4 325 961. The compounds of the Formula I may also be advantageously combined with an $H_1$ or $H_2$-receptor antagonist, such as benadryl, dramamine, histadyl, phenergan, terfenadine, acetamezole, cimetidine, ranitidine, famotidine, aminothiadiazoles disclosed in EP-A-0 040 696 and those disclosed in US Patent Specifications US-A-4 283 408, US-A-4 362 736, US-A-4 394 508; and pending US Patent Application No. 301 616, filed 14 September 1981. The pharmaceutical compositions may also contain a $K^+/H^+$ ATPase inhibitor such as omeprazole, which is disclosed in US-A-4 255 431. Another useful pharmaceutical composition comprises a compound of Formula I in combination with serotonin antagonists such as methysergide or the serotonin antagonists disclosed in Nature, vol. 316, pages 126-131, 1985.

Compounds of the present invention can be prepared according to the following methods.

METHOD A

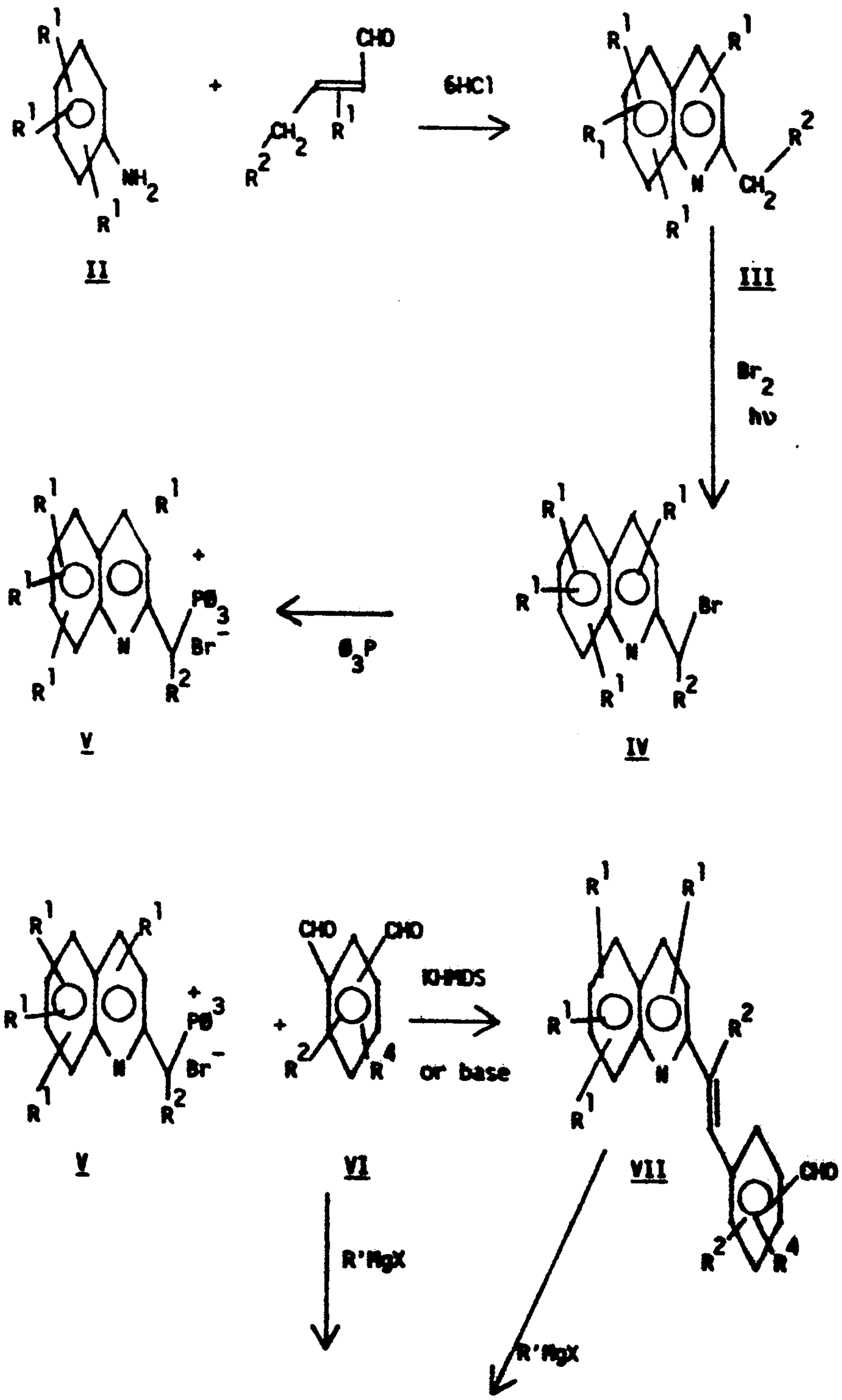


II III IV V V VI VII

METHOD A (Cont.)

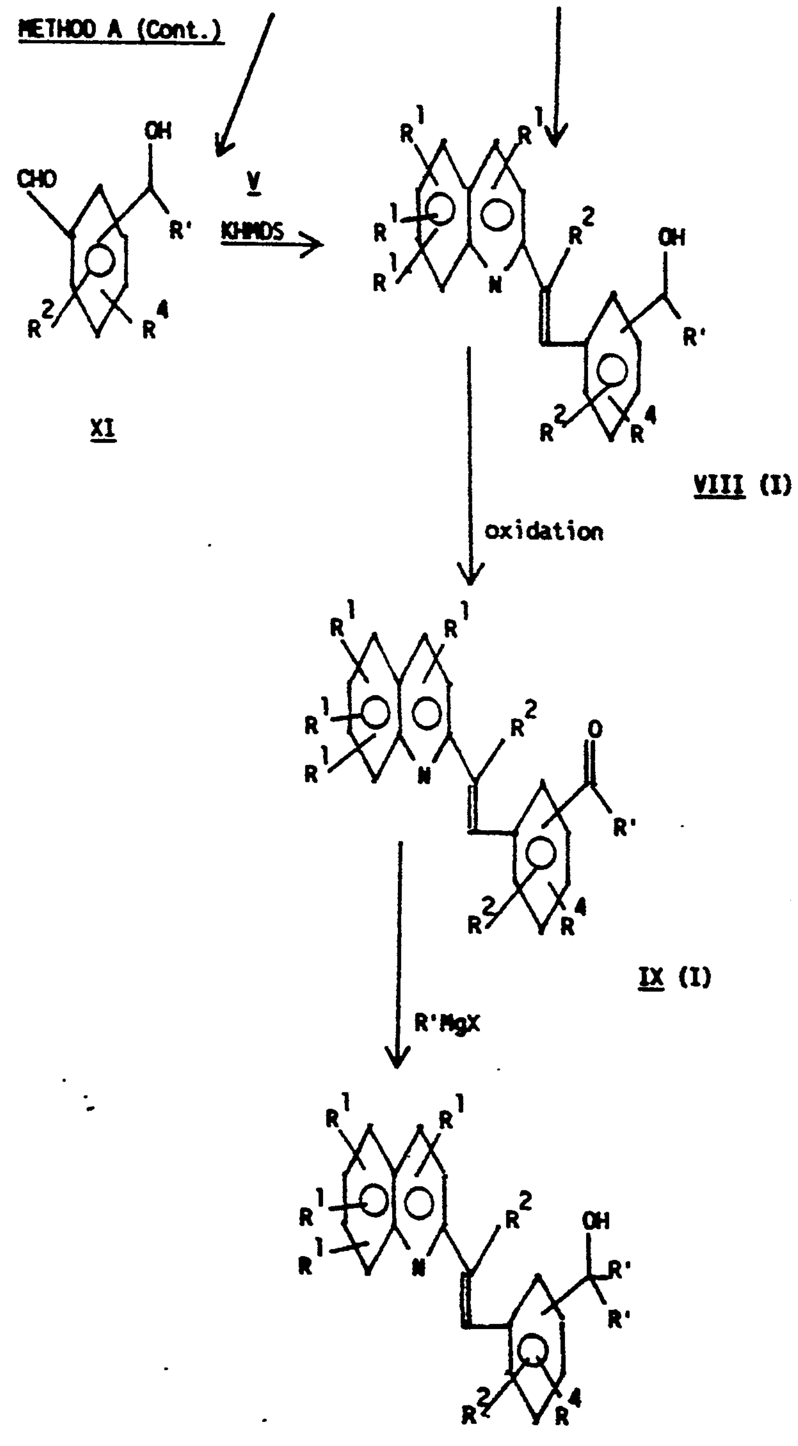


XI

VIII (I)

IX (I)

X (I)

$R' = -CR^2R^2-(CR^2R^2)_{m-1}-(CR^2R^4)_m-CR^2_2R^4$

X = Cl, Br or I

METHOD B

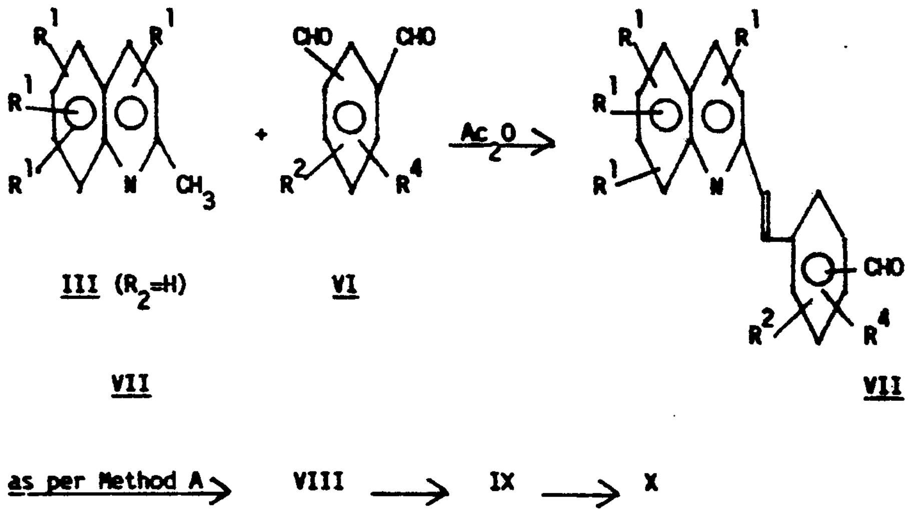


III ($R_2$=H) VI VII VII

VIII IX X

METHOD C

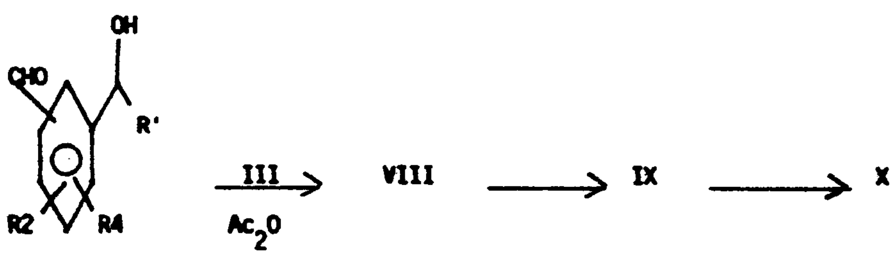


VIII IX X

XI

METHOD A

Referring to Method A, an aniline derivative of Formula II is reacted by heating with a crotonaldehyde and a strong mineral acid such as aqueous hydrochloric acid to provide the substituted quinaldine derivative of structure III. When II is unsymmetrical two regioisomers of II may be obtained. The products are purified by precipitation of the zinc chloride adducts or by standard chromatographic techniques. Derivative III is treated with a halogen source such as $Br_2$ in the presence of light to afford quinaldine IV. This product is treated with triphenylphosphine in an inert solvent such as toluene with heating to give phosphonium salt V.

A Wittig reagent of general formula V is reacted with a strong base such as a n-butyllithium or KHMDS (potassium hexamethyldisilazide) and then with a dialdehyde of general formula VI to provide the olefin of general formula VII. Reaction of VII with a carbanionic reagent such as a Grignard reagent R'MgX or an alkyllithium reagent R'Li in an inert solvent provides the alcohols of general formula VIII. Oxidation of VIII with an oxidizing agent such as $MnO_2$ provides the ketones of general formula IX. IX may be further reacted with R'MgX or R'Li to provide the alcohols of general formula X.

Alternatively, a dialdehyde VI can be first reacted with R'MgX or R'Li to provide XI and then XI reacted with the ylid derived from V to provide VIII.

Compounds VIII, IX and X are all representative of the Formula I compounds.

METHOD B

A quinaldine derivative of general formula III is reacted with a dialdehyde VI in the presence of a dehydrating agent such as acetic anhydride to provide the olefin VII which is further elaborated to VIII, IX and X as described in Method A.

METHOD C

4-(2-(Quinolin-2-yl)ethenyl)benzaldehyde

Alternatively, an aldehyde alcohol of general formula XI is reacted with III in the presence of dehydrating agent such as acetic anhydride to provide VIII and then IX and X as described above.

This invention is further defined by reference to the following examples, which are intended to be illustrative and not limiting.

All temperatures are in degrees Celsius.

EXAMPLE 1

4-(2-(Quinolin-2-yl)ethenyl)benzaldehyde

To a suspension of quinolin-2-ylemethyltriphenylphosphonium bromide (10 g) in THF (200 ml) at -78° was added potassium hexamethyldisilizane (34 ml of 0.67 M solution). The suspension was stirred 1 hour at -78° and then added to a solution of terephtaldehyde (4.4 g) in THF (100 ml). The resulting brown solution was stirred 2 hours at -78°, quenched with $NH_4OAc$ buffer (500 ml) and extracted with ethyl acetate. The organic layer was dried and evaporated. Flash chromatography of the residue afforded the title compound; m.p. 107-108°.

EXAMPLE 2

2-[2-(4-(1-Hydroxyhexyl) phenyl) ethenyl quinoline

To a solution of the aldehyde (Example 1) (254 mg) in THF (12 ml) was added pentylmagnesium bromide (1.2 ml of a 1M solution) at -78°. After 1 hour, another 1 ml of pentylmagnesium bromide was added. After a further 1 hour, the reaction was quenched with $NH_4OAc$ (buffer) and extracted with ethylacetate. The ethylacetate layer was dried ($NA_2SO_4$), filtered and evaporated. Flash chromatography of the residue using from 20 to 40% ethylacetate in hexane afforded the title compound; m.p. 140-142°.

Anal. Calc'd for $C_{18}H_{13}NO$:

C, 83.30; H, 7.60; N, 4.20

Found: C, 83.90; H, 8.45; N, 4.17

EXAMPLE 3

2-(2-(4-1-Hydroxyhexyl) phenyl) vinyl quinoline

To a solution of aldehyde (Example 1) (1 g) in THF (33 ml) at -78° was added methyllithium (3.3 ml of 1.3M). The reaction mixture was stirred 30 minutes at -78°, quenched with 25% $NH_4OAc$ and extracted with ethylacetate. The organic layer was dried and evaporated. Flash chromatography of the residue using 25 to 30% ethylacetate hexane afforded the title compound; m.p. 155-157°C.

Anal. Calc'd $C_{18}H_{13}NO$:

C, 82.87; H, 6.22; N, 5.09

Found: C, 82.77; H, 6.20; N, 5.01

EXAMPLE 4

4-(2-(Quinolin-2-yl) ethenyl) acetophenone

To solution of the alcohol (Example 3) (440 mg) in ethylacetate (50 ml) was added $MnO_2$ (550 mg) in 3 portions over 1 hour. The reaction mixture was stirred 5 hours, filtered through a pad of $SiO_2$ and evaporated. Recrystallization of the residue from 1:1 ethylacetate/hexane afforded the title compound; m.p. 160-161°

Anal. Calc'd for $C_{19}H_{15}NO$:

C, 83.50; H, 5.53; N, 5.13

Found; C, 83.59; H, 5.74; N, 4.94

EXAMPLE 5

3-(2-(Quinolin-2-yl) ethenyl) benzaldehyde

Using the procedure described in Example 1 but substituting isophtaldehyde for terephtaldehyde there was obtained the title compound; m.p. 88°-89°. Anal. Calc'd for $C_{18}H_{13}NO$:

C, 83.38; H, 5.05; N, 5.40

Found: C, 83.26; H, 5.29; N, 5.23

EXAMPLE 6

3-(2-(Quinolin-2-yl) ethenyl) benzyl alcohol

To a solution of the aldehyde (Example 5) (408 mg) in ethanol (30 ml) was added sodium borohydride (110 mg). Ammonium acetate (100 ml of 25% solution) was added. "the mixture was extracted with ethylacetate

(200 ml), dried and evaporated. The residue was purified by flash chromatography using 30% ether in hexane. Recrystallization from ether/hexane afforded the title compound; m.p. 131-132°.
Anal. Calc'd for $C_{18}H_{15}NO$:
C, 82.74; H, 5.79; N, 5.36
Found: C, 82.51; H, 5.53; N, 5.23

EXAMPLE 7

2-[2-(3-(1-Hydroxyethyl) phenyl) ethenyl]quinoline

Using the procedure of Example 3 but substituting the aldehyde from Example 5 for the aldehyde from Example 1 there was obtained the title compound; m.p. 113-114°.
Anal. Calc'd for $C_{19}H_{17}NO$:
C, 82.87; H, 6.22; N, 5.09
Found: C, 82.57; H, 6.28; N, 5.14

EXAMPLE 8

3-[2-(Quinolin-2-yl) ethenyl)acetophenone

Using the procedure of Example 4 but substituting the alcohol from Example 7 for the alcohol from Example 3 there was obtained the title compound; m.p. 129-130°.
Anal. Calc'd for $C_{19}H_{15}NO$:
C, 83.50; H, 5.53; N, 5.13
Found: C, 83.30; H, 5.68; N, 5.16

EXAMPLE 9

2-[2-(3-(1-Hydroxy-l-methylethyl) phenyl) ethenyl]quinoline

Using the procedure of Example 3 but substituting the ketone from Example 8 for the aldehyde from Example 1 there was obtained the title compound; m.p. 150-153°.
Anal. Calc'd for $C_{20}H_{19}NO$:
C, 83.01; H, 6.62: N, 4.84
Found C, 82.87: H, 6.75; N, 4.79

EXAMPLE 10

2-[2-(3-(1-Hydroxypropyl)phenyl)ethenyl quinoline

Using the procedure of Example 2 but substituting the aldehyde from Example 5 for the aldehyde from Example 1 and ethylmagnesium bromide for pentylmagnesium bromide there was obtained the title compound; m.p. 125-126°.
Anal. Calc'd for $C_{20}H_{19}NO$:
C, 83.01; H, 6.62; N, 4.84
Found: C, 82.91; H, 6.62; N, 4.79

EXAMPLE 11

7-Bromo-2-[2-(3-(1-hydroxypropyl)phenyl)ethenyl)] quinoline

Step 1: Preparation of 3-(2-(7-bromoquinolin-2-yl)ethenyl)benzaldehyde

A suspension of isophtaldehyde (10 g), 7-bromoquinaldine (16.6 g) and acetic anhydride (75 ml) was heated at 125° for 48 hours. The reaction mixture was cooled, diluted with ether (100 ml) and filtered to give the title compound which was used as is for the next step.

Step 2:

Using the procedure of Example 2 but substituting the aldehyde from Example II, Step I, for the aldehyde from Example 1 and ethylmagnesium bromide for pentylmagnesium bromide there was obtained the title compound; m.p. 106°-107°.
Anal. Calc'd for $C_{20}H_{18}BrNO$:
C, 65.23; H, 4.93; N, 3.80
Found: C, 65.26; H, 5.22: N, 3.66

**Claims**

1. A compound of the formula:

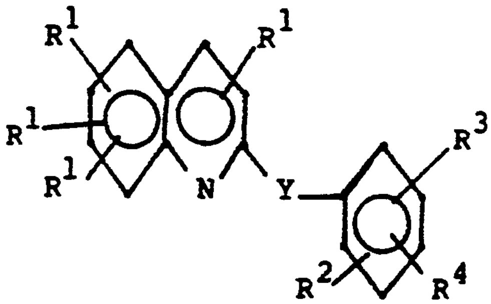

I

in which:

is $-(CR^2=CR^2)_n$-or $-(C\equiv C)_n-$;

$R^1$ is H, halogen, $C_1$-$C_8$ alkyl, $C_2$-$C_8$ alkenyl, $C_2$-$C_8$ alkynyl, $-CR_3$, $OR^2$, $-SR^2$, $-SOR^2$, $-SO_2R^2$, $-NR^2R^2$, -CHO, $-COOR^2$, $-(C=O)R^2$, $-C(OH)R^2R^2$, -CN, $-NO_2$, substituted or unsubstituted phenyl, substituted or unsubstituted benzyl, or substituted or unsubstituted phenethyl;

$R^2$ is H, $C_1$ -$C_8$ alkyl, $C_2$-$C_8$ alkenyl, $C_2$-$C_8$ alkynyl, $-CF_3$, substituted or unsubstituted phenyl, substituted or unsubstituted benzyl, or substituted or unsubstituted phenethyl;

$R^3$ is $-(A)_m-(CR^2R^2)_m-(CR^2R^4)_m-CR^2R$ , with the proviso that $R^3$ is not CHO when it is para to Y;

$R^4$ is H, halogen, $-NO_2$, $-OR^2$, $-SR^2$, $NR^2R^2$, or $C_1$-$C_8$ alkyl, or $(R^4)_2WO=$;

A is $=C=O$ or $CR^2(OR^6)$;

$R^6$ is H, $C_1$-$C_6$ alkyl, $-(C=O)R^7$, unsubstituted phenyl or unsubstituted benzyl;

$R^7$ is H, $C_1$-$C_8$ alkyl, $C_2$-$C_8$ alkenyl, $C_2$-$C_8$ alkynyl, $-CF_3$, or unsubstituted phenyl, benzyl, or phenethyl;

each m is 0 or integer of from 1 to 8, provided that at least one m is not 0;

and n is 1 or 2; provided that where two or more of any variable are present, each is the same as or different from the other(s), or a compound that is a pharmaceutically acceptable salt thereof.

2. A compound as claimed in claim 1 of the formula:

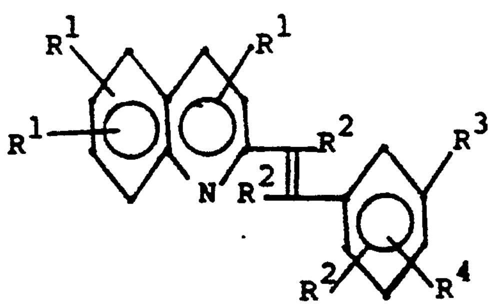

Ia

in which $R^1$ is H, halogen, $CH_3$, $-CF_3$, or $SCF_3$; $R^2$ is H, $C_{1-3}$ alkyl, $C_{2-3}$alkenyl, or $-CF_3$; and $R^3$ to $R^7$, m and A are as defined in claim 1, or a compound that is a pharmaceutically acceptable salt thereof.

3. A compound as claimed in claim 1 of the formula:

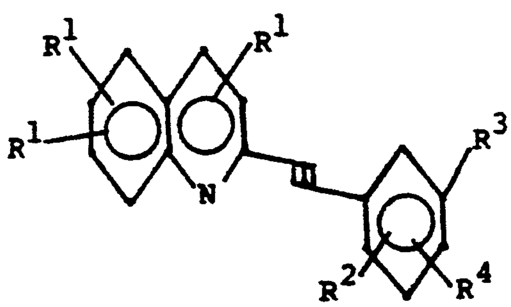

Ib

in which $R^1$ to $R^7$, m and A are as defined in claim 2, or a compound that is a pharmaceutically acceptable salt thereof.

4. 2-[2-(4-(1-Hydroxyhexyl)phenyl)ethenyl] quinoline;

2-(2-(4-(1-hydroxymethyl)phenyl)vinyl quinoline;

4-(2-(quinolin-2-yl))ethenyl) acetophenone;
3-(2-quinolin-2-yl)ethenyl)benzaldehyde;
3-(2-quinolin-2-yl))ethenyl)benzyl alcohol;
2-[2-(3-(1-hydroxyethyl)phenyl)ethenyl] quinoline;
3-(2-(quinolin-2-yl)ethenyl)acetophenone;
2-[2-(3-(1-hydroxy-1-methylethyl)phenyl)ethenyl] quinoline;
2-[2-(3-(1-hydroxypropyl)phenyl)ethenyl quinoline; or
7-bromo-2-[2-(3-(1-hydroxypropyl)phenyl)ethenyl] quinoline;

5. A pharmaceutical composition useful in antagonizing leukotriene action in mammals comprising a compound as claimed in any one of claims 1 to 4 in an amount effective as a leukotriene antagonist and a pharmaceutically acceptable carrier.

6. A composition as claimed in claim 5 additionally comprising an effective amount of a second active ingredient that is a non-steroidal anti-inflammatory drug; a peripheral analgesic agent; cyclooxygenase inhibitor; a leukotriene antagonist; a leukotriene inhibitor; an $H_2$-receptor antagonist; an antihistaminic agent; a prostaglandin antagonist or a thromboxane antagonist.

7. A composition according to claim 6, in which the second active ingredient is a non-steroidal anti-inflammatory drug.

8. A compound as claimed in any one of claims 1 to 4 for use in preventing the synthesis, the action, or the release of SRS-A or leukotriene $D_4$ in mammals.

9. A compound as claimed in any one of claims 1 to 4 for use in inducing cytoprotection in mammals..

10. A compound as claimed in any one of claims 1 to 4 for use in inflammatory diseases of the eye in mammals.